(19) Europäisches Patentamt
European Patent Office
Office européen des brevets

(11) Numéro de publication: **0 020 266**
**A1**

(12) # DEMANDE DE BREVET EUROPEEN

(21) Numéro de dépôt: **80400752.4**

(22) Date de dépôt: **28.05.80**

(51) Int. Cl.³: **C 07 D 307/85**, C 07 D 333/70, A 61 K 31/34, A 61 K 31/38 // C07D407/12, C07D409/12

(30) Priorité: **29.05.79 FR 7913613**

(43) Date de publication de la demande: **10.12.80 Bulletin 80/25**

(84) Etats contractants désignés: **AT BE CH DE FR GB IT LI LU NL SE**

(71) Demandeur: **ANVAR Agence Nationale de Valorisation de la Recherche, 13, rue Madeleine Michelis, F-92522 Neuilly-sur-Seine (FR)**

(72) Inventeur: **Royer, René Richard, 12 rue du Pont des Loges, Paris (FR)**
Inventeur: **Rene, Loic Jean Alexis, Institut Curie Section Biologie, 26 rue d'Ulm 75231 Paris Cedex 05 (FR)**
Inventeur: **Aurousseau, Michel Emile Marie, 104 boulevard Arago, Paris (FR)**

(74) Mandataire: **Casanova, André CABINET ARMENGAUD JEUNE CASANOVA, AKERMAN, LEPEUDRY et al, 23 boulevard de Strasbourg, F-75010 Paris (FR)**

(54) **Nouveaux dérivés des cyano-2 hydroxy-3 benzofuranne et benzo(b)thiophène, leur préparation et leur application en thérapeutique.**

(57) Composé de formule

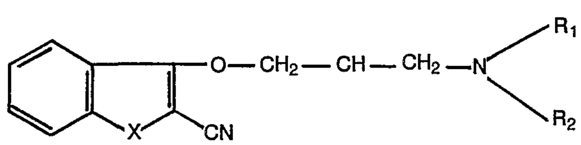

dans laquelle X représente un atome d'oxygène ou de soufre, $R_1$ et $R_2$ représentent chacun un atome d'hydrogène ou un groupe alkyle inférieur, ou bien $R_1$ et $R_2$ forment avec l'atome d'azote auxquels ils sont attachés un groupe pyrrolidino, pipéridino ou morpholino, ainsi que leurs sels d'addition à des acides pharmaceutiquement acceptables.

Médicaments $\beta$-bloqueurs.

EP 0 020 266 A1

Nouveaux dérivés des cyano-2 hydroxy-3 benzofuranne et benzo /‾b_7 thiophène, leur préparation et leur application en thérapeutique.

La présente invention a pour objet de nouveaux composés, dérivés des cyano-2 hydroxy-3 benzofuranne et benzo/‾b7thiophène, leur préparation et leur application en thérapeutique.

Les adrénolytiques de synthèse ont connu depuis longtemps un grand développement. Leur structure au départ était inspirée de l'épinéphirine (adrénaline), mais s'est progressivement modifiée pour arriver à des phénoxy-hydroxy-propylamines, qui manifestent des effets β-bloquants. On peut citer en particulier le propranolol ou isopropylamino-1 (naphtyl-1 oxy)-3 propanol-2 (brevet Belge n° 640 312 ou brevet des Etats-Unis d'Amérique n° 3 337 628).

Or les Demandeurs ont trouvé des analogues hétérocycliques de cette structure, possédant des propriétés β-bloquantes comparables tout en ne présentant pas certaines propriétés spécifiques indésirables dans les domaines cardiaque et bronchique.

Les composés de l'invention répondent à la formule générale

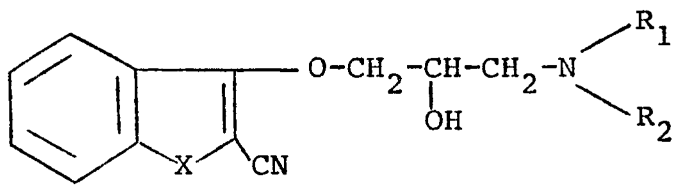

(I)

dans laquelle

X représente un atome d'oxygène ou de soufre,

$R_1$ et $R_2$ représentent chacun un atome d'hydrogène ou un groupe alkyle inférieur, ou bien $R_1$ et $R_2$ forment avec l'atome d'azote auxquels ils sont attachés un groupe pyrrolidino, pipéridino ou morpholino,

ainsi que leurs sels d'addition à des acides pharmaceutiquement acceptables.

On entend par groupe alkyle inférieur un groupe alkyle ayant de 1 à 6 atomes de carbone. On préfère selon l'invention les dérivés monoalkylamino ($R_1$ = alkyle, surtout tert-butyle, $R_2$ = H).

BAD ORIGINAL

Les composés de formule I selon l'invention peuvent être préparés par condensation d'éthers époxy-2,3 propyliques de formule

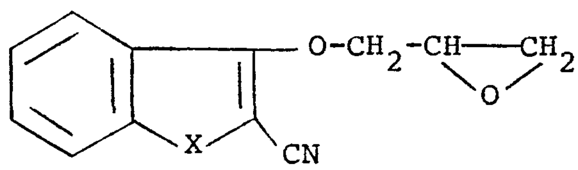

(II)

dans laquelle X est un atome d'oxygène ou de soufre, avec une amine appropriée $HNR_1R_2$ (III), les symboles $R_1$ et $R_2$ ayant les mêmes significations que ci-dessus dans la formule (I).

Cette réaction de condensation est avantageusement effectuée dans un solvant polaire, en particulier l'éthanol.

Les éthers époxy-2,3 propyliques de formule II sont préparés par traitement du cyano-2 hydroxy-3 benzofuranne, ou le cas échéant du cyano-2 hydroxy-3 benzo[b] thiophène, avec l'épichlorhydrine du glycol, en présence de carbonate de potassium dans le diméthylformamide. Les cyano-2 hydroxy-3 benzofuranne et benzo[b]thiophène sont connus (cf : M. PESSON et M. JOANNIC, Chem. Abstr. 1970, 72, 90264 ; K. GOERLITZER, Arch. Pharm. 1975, 308, 700 ; R. BRYANT et D.L. HASLAM, J. Chem. Soc. 2361, (1965).

L'exemple suivant illustre la préparation des composés de l'invention.

EXEMPLE :

a) Ethers époxy-2,3 propyliques

On chauffe à l'ébullition, au reflux, pendant 10 minutes, une solution à 10 % dans le diméthylformamide des quantités équimolaires d'épichlorhydrine du glycol et de cyano-2 hydroxy-3 benzofuranne, en présence de la quantité correspondante de carbonate de potassium. On verse sur de la glace, épuise au benzène, poursuit selon l'usage et recristallise dans l'éther de pétrole (ou dans un mélange d'éther de pétrole et de benzène) pour isoler le cyano-2 (époxy-2,3 propyl)-3 benzofuranne (formule II ; X = 0) avec un rendement de 31 % ; F = 87°.

En opérant de la même manière que ci-dessus et en partant de cyano-2 hydroxy-3 benzo[b]thiophène, on obtient avec un rendement de 55 % le cyano-2 (époxy-2,3 propyl)-3 benzo[b]thiophène (formule II ; X = S) ; F = 75°.

b) <u>Ethers alkylamino-3 hydroxy-2 propyliques</u> (formule I).

On maintient à l'ébullition, au reflux, pendant 2 heures, une solution à 10 % dans l'éthanol d'éther époxy-2,3 propylique (II) obtenu ci-dessus et d'un excès de l'amine voulue de formule III. On élimine le solvant et l'amine qui n'a pas réagi par chauffage sous pression réduite, puis recristallise le résidu dans l'éther de pétrole.

Dans le tableau I ci-dessous sont rassemblés les composés de formule (I), le rendement de la synthèse et leur point de fusion. Tous les nouveaux composés étudiés ici présentent des analyses centésimales pour C, H, N et, le cas échéant, S, conformes à ± 0,2 à la théorie.

TABLEAU I

| Composé n° | X | $-NR_1R_2$ | Rdt* | F (°C) | Maléate, F (°C) |
|---|---|---|---|---|---|
| 1 | O | $-NH-C(CH_3)_3$ | 75 | 88 | 182 |
| 2 | S | $-NH-C(CH_3)_3$ | 85 | 97 | 206 |
| 3 | O | $-NH-n-C_3H_7$ | 78,5 | 99 | 124 |
| 4 | S | $-NH-n-C_3H_7$ | 87 | 100 | 161 |
| 5 | S | 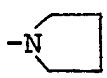 | 76 | 103 | 171 |
| 6 | O | 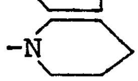 | 57 | 70 | 136 |
| 7 | S | 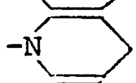 | 82 | 97 | 159 |
| 8 | O | 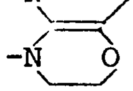 | 86 | 84 | 138 |
| 9 | S | 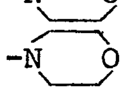 | 75,5 | 105 | 163 |

* Par rapport à l'éther époxy-2,3 propylique correspondant.

Les composés de l'invention ont été soumis à des essais pharmacologiques qui ont permis de mettre en évidence leurs propriétés β-bloquantes. Les composés ont été administrés sous forme de maléates.

L'activité β-lytique a été recherchée au niveau des récepteurs $\beta_1$-cardiaques en déterminant l'antagonisme des effets de l'isoprénaline sur l'oreillette isolée de cobaye, selon la méthode de Giudicelli, Schmitt et Boissier (J. Pharmacol. Exper. Thérapeut., 1969, 168, 116). Les essais ont été effectués sur cinq préparations par produit, à une concentration unique de $1.10^{-7}$ g/$cm^3$. Le composé n° 2 s'est montré suffisamment efficace, vis-à-vis aussi bien de l'effet inotrope + de l'isoprénaline que de son effet chronotrope +. C'est pourquoi il a été soumis à une étude complémentaire à des doses plus faibles et comparé, pour référence, au propranolol sur une même préparation.

Afin de ne pas surcharger inutilement les tableaux suivants, seuls les résultats correspondant au composé n° 2 sont indiqués. Les résultats rassemblés dans le tableau II ci-dessous montrent qu'à de faibles concentrations, le composé 2 a une activité $\beta_1$-stimulante proche de celle du propranolol (p).

Voir tableau II page suivante.

TABLEAU II

Action antagoniste de l'effet $\beta_1$-stimulant de l'isoprénaline

| Produit n° | Dose en mole/$cm^3$ | Effet chronotrope + de l'isoprénaline | | | Effet inotrope + de l'isoprénaline | | |
|---|---|---|---|---|---|---|---|
| | | Concentration active (a) | | Index d'activité (b) | Concentration active (c) | | Index d'activité (b) |
| | | Avant produit | Après produit | | Avant produit | Après produit | |
| 2 | $1.10^{-10}$ | $5,4.10^{-9}$ | $3,8.10^{-8}$ | 7 | $1,2.10^{-8}$ | $7,6.10^{-8}$ | 6,5 |
| 2 | $5.10^{-10}$ | $2,2.10^{-8}$ | $1,5.10^{-7}$ | 7 | $7,4.10^{-9}$ | $2,5.10^{-8}$ | 3,5 |
| propranolol | $5.10^{-10}$ | $4,9.10^{-9}$ | $1,8.10^{-7}$ | 37 | $1.10^{-9}$ | $5,8.10^{-8}$ | 58 |
| 2 | $5.10^{-10}$ | | $1,8.10^{-7}$ | 37 | | $5,4.10^{-8}$ | 54 |

(a) Concentration donnant une augmentation de 30 battements par min.

(b) Déterminé par le rapport des concentrations actives après et avant l'addition du produit.

(c) Concentration donnant une augmentation de 10 mm de l'amplitude du tracé.

L'action $\beta_2$-adrénolytique au niveau bronchique a été étudiée chez le cobaye, à raison de cinq animaux par essai, en recherchant l'inhibition de la protection exercée par l'isoprénaline vis-à-vis du bronchospasme à la sérotonine, selon la méthode de Konzett et Roessler (Arch. Exper. Pathol. Pharmakol., 1940, 195, 71) modifiée par Halpern. Arch. Internat. Pharmacodyn. 1942, 68, 339 .

Les composés sont administrés par voie i.v. à la dose de 250 µg/kg.

Les résultats rapportés sur le tableau III ci-dessous révèlent que le composé 2 présente l'avantage sur le propranolol (p) de n'exercer qu'un blocage très fugace des récepteurs $\beta_2$-bronchiques. Dans ce tableau les nombres entre parenthèses représentent les pourcentages d'inhibition de l'isoprénaline.

Voir tableau III page suivante.

TABLEAU III

Action antagoniste de l'effet $\beta_2$-stimulant bronchique de l'isoprénaline

| Produit | Moyenne de l'amplitude | | | | | | |
|---|---|---|---|---|---|---|---|
| | Avant administration | Après administration, au bout de | | | | | |
| | | 1 min | 8 min | 16 min | 30 min | 60 min | 50 min |
| P | 21 ± 3,3 | 23,2 ± 1,8 (100) | 17,8 ± 1,6 (85) | 9 ± 2,4 (43) | 4 ± 2,0 (19) | 1,2 ± 1,2 (6) | 0 |
| 2 | 24,9 ± 3,9 | 20,4 ± 1,4 (82) | 3,8 ± 2,4 (15) | 0,6 ± 0,6 (2) | 0 | 0 | |

En conclusion, bien que les composés de l'invention n'aient pas des propriétés $\beta_1$-bloquantes supérieures à celles du propranolol, le fait qu'ils soient sans effet notable sur les récepteurs $\beta_2$-bronchiques et qu'ils n'exercent pas d'action stimulante cardiaque spécifique incite à les retenir pour une utilisation en thérapeutique.

Etant donné l'activité pharmacologique des composés de l'invention, leur application en thérapeutique peut être d'une manière générale celle des $\beta$-bloquants. Ils peuvent être utilisés plus particulièrement dans des cas d'angine de poitrine, d'hypertension artérielle, et aussi de troubles du rythme cardiaque.

Ils peuvent être administrés par voie orale à des doses quotidiennes allant de 100 à 600 mg et, dans les cas sévères, par voie I.V. à raison de 1 à 15 mg.

REVENDICATIONS DE BREVET

1.- Composés répondant à la formule

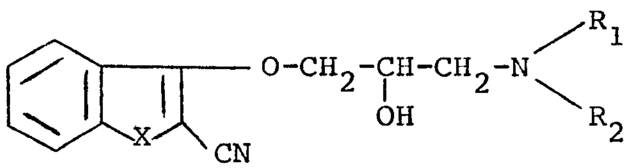

(I)

dans laquelle X représente un atome d'oxygène ou de soufre, $R_1$ et $R_2$ représentent chacun un atome d'hydrogène ou un groupe alkyle inférieur, ou bien $R_1$ et $R_2$ forment avec l'atome d'azote auxquels ils sont attachés un groupe pyrrolidino, pipéridino ou morpholino, ainsi que leurs sels d'addition à des acides pharmaceutiquement acceptables.

2.- Composés selon la revendication 1, répondant à la formule (I) dans laquelle $R_1$ est un groupe alkyle inférieur et $R_2$ est un atome d'hydrogène.

3.- Le cyano-2 (hydroxy-2 tert-butylamino-3 propoxy)-3 benzo[b]thiophène et son maléate.

4.- Procédé de préparation des composés selon la revendication 1, procédé caractérisé en ce que l'on condense un éther époxy-2,3 propylique de formule

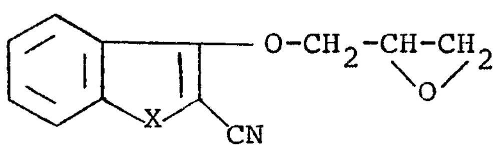

(II)

dans laquelle X est un atome d'oxygène ou de soufre, avec une amine $HNR_1R_2$, les symboles $R_1$ et $R_2$ ayant les mêmes significations que dans la formule I.

5.- Médicament comprenant l'un des composés spécifiés dans l'une quelconque des revendications 1 à 3.

6.- Médicament comprenant le composé spécifié dans la revendication 3.

7.- Composition pharmaceutique contenant l'un des composés selon l'une quelconque des revendications 1 à 3 et des excipients pharmaceutiques usuels.

Office européen des brevets

# RAPPORT DE RECHERCHE EUROPEENNE

0020266

Numéro de la demande

EP 80 40 0752

## DOCUMENTS CONSIDERES COMME PERTINENTS

| Catégorie | Citation du document avec indication, en cas de besoin, des parties pertinentes | Revendication concernée |
|---|---|---|
| | FR - A - 2 068 430 (LAB. BELLON)<br>* Pages 13,14; revendications *<br>-- | 1,5-7 |
| | FR - A - 2 137 901 (KAKENYAKU KAKO)<br>* Pages 29-31; revendications *<br>---- | 1-7 |

**CLASSEMENT DE LA DEMANDE (Int. Cl. 3)**

C 07 D 307/85
333/70
A 61 K 31/34
31/38//
C 07 D 407/12
C 07 D 409/12

**DOMAINES TECHNIQUES RECHERCHES (Int. Cl. 3)**

C 07 D 307/85
C 07 D 333/70

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 21-08-1980 | ALLARD |